# EUROPÄISCHE PATENTSCHRIFT

(11) **EP 0 795 567 B1**
(45) Veröffentlichungstag und Bekanntmachung des Hinweises auf die Patenterteilung: **12.06.2002**
(21) Anmeldenummer: 97104190.0
(22) Anmeldetag: 12.03.1997
(51) Int. Cl.: C08F 226/06, A61K 7/00

(54) **Verfahren zur Herstellung von wasserlöslichen Copolymerisaten aus wenigstens einem wasserlöslichen N-Vinyllactam und wenigstens einem hydrophoben Comonomeren**
Process for preparing hydrosoluble copolymers of at least one hydrosoluble n-vinyllactam and at least one hydrophobic comonomer
Procédé de préparation de copolymères solubles dans l'eau d'au moins un n-vinyllactam soluble dans l'eau et d'au moins un comonomère hydrophobe

(30) Priorität: 13.03.1996 DE 19609864
(43) Veröffentlichungstag der Anmeldung: 17.09.1997
(73) Patentinhaber: BASF AKTIENGESELLSCHAFT, 67056 Ludwigshafen (DE)
(72) Erfinder: Blankenburg, Rainer, Dr., 67067 Ludwigshafen (DE); Kothrade, Stephan, Dr., 67117 Limburgerhof (DE); Sanner, Axel, Dr., 67227 Frankenthal (DE)
(74) Vertreter: Kinzebach, Werner, Dr.

(56) Entgegenhaltungen:
- EP-A- 0 215 379
- WO-A-93/18073

## Beschreibung

Die vorliegende Erfindung betrifft ein Verfahren zur Herstellung wasserlöslicher Copolymerisate aus wenigstens einem wasserlöslichen N-Vinyllactam und wenigstens einem hydrophoben Comonomer durch radikalische Polymerisation der Monomeren in einem wässrigen Lösungsmittel, sowie die nach dem Verfahren erhältlichen Copolymerisate und deren Verwendung.

Die Herstellung von Copolymerisaten aus N-Vinyllactamen und hydrophoben Comonomeren durch radikalische Polymerisation ist bekannt. Für viele Anwendungszwecke sind Copolymerisate erwünscht, die in Wasser klar löslich sind, d.h. der FNU-Wert einer 5 Gew.-%igen Lösung soll ≤ 20 sein. Die Herstellung derartiger Copolymerisate erfolgt in einem organischen Lösungsmittel, beispielsweise einem Alkohol oder in einem Gemisch aus Wasser und organischem Lösungsmittel mit einem hohen Anteil an Lösungsmittel. So beschreibt die US 5,395,904 die Polymerisation von Vinylpyrrolidon und Vinylacetat nach der Zulaufmethode. Es wird ein alkoholisches Lösungsmittel verwendet, das bis zu 50 Gew.-% Wasser enthalten kann. Die Polymere sind wasserlöslich, weisen jedoch nur ein geringes Molekulargewicht im Bereich von 6000 bis 50000 g/mol auf, entsprechend einem K-Wert (nach H. Fikentscher, Cellulose-Chemie, Bd. 13, 1932, S. 58-64) von 10 bis 40.

Die Copolymerisation in wässriger Phase hätte jedoch deutliche Vorteile gegenüber den herkömmlichen Vorgehensweisen. Bei der radikalischen Copolymerisation greift Wasser im Unterschied zu Alkoholen nicht als Regler ein, so daß Copolymere mit hohen Molekulargewichten (K-Werte > 50) zugänglich wären. Es bestünde dann die Möglichkeit, das Molekulargewicht durch Einsatz geeigneter Regler gezielt einzustellen. Auch ließen sich durch einen bewußten Verzicht auf organische Lösungsmittel die Produktionskosten bislang bestehender Produktionsverfahren verringern und deren Umweltverträglichkeit verbessern. Copolymerisationen in Wasser haben jedoch bisher nicht die gewünschten klar wasserlöslichen Copolymerisate ergeben.

So beschreiben die US 4,520,179 und US 4,554,311 die Polymerisation von Vinylpyrrolidon und Vinylacetat mit t-Butylperoxypivalat als Initiator in Wasser oder Wasser/Alkohol-Mischungen. Der dort verwendete Initiator erlaubt die Herstellung von Copolymerisaten mit enger Molekulargewichtsverteilung, er führt jedoch nicht zu wasserlöslichen Produkten mit einem FNU-Wert ≤ 20.

Die DE-OS 22 18 935 beschreibt die Copolymerisation von N-Vinylpyrrolidon mit verschiedenen wasserlöslichen und wasserunlöslichen Comonomeren. Dabei werden wasserunlösliche Initiatoren verwendet, die in Form einer feinteiligen Suspension in einer wäßrigen Lösung der Copolymerisate zur Anwendung kommen. Dies führt im Falle der wasserunlöslichen Comonomere jedoch ebenfalls nicht zu den gewünschten wasserlöslichen Copolymeren mit einem FNU-Wert ≤ 20.

Die WO 93/18073 beschreibt ein Verfahren zur Herstellung von Copolymeren aus monoethylenisch ungesättigten Monomeren mit Säuregruppen und N-Vinyllactamen durch Copolymerisation der Monomere in Gegenwart von Radikale bildenden Polymerisationsinitiatoren im Sinne einer Lösungspolymerisation in Wasser bei einem pH-Wert von mindestens 6, wobei zur Einstellung des pH-Werts der wässrigen Reaktionslösung eine Alkalimetall- und/oder Erdalkalimetallbase eingesetzt wird.

Die EP-A 215 379 beschreibt die Herstellung von Polyvinylpyrrolidon in wässriger Lösung mit Wasserstoffperoxid als Starter, wobei der pH-Wert der Reaktionslösung mit Hilfe von NaOH, KOH, deren Carbonat oder Bicarbonat zwischen 7 und 11 gehalten wird.

Der vorliegenden Erfindung liegt daher die Aufgabe zugrunde, ein Verfahren zur Herstellung von klar wasserlöslichen Copolymerisaten aus wenigstens einem hydrophilen N-Vinyllactam und wenigstens einem hydrophoben Comonomeren durch radikalische Copolymerisation in einem wässrigen Lösungsmittel bereitzustellen.

Die Aufgabe wird erfindungsgemäß gelöst durch ein Verfahren zur Herstellung klar wasserlöslicher Copolymerisate, die aufgebaut sind aus 40 bis 90 Gew.-% wenigstens eines wasserlöslichen N-Vinyllactams der allgemeinen Formel I, 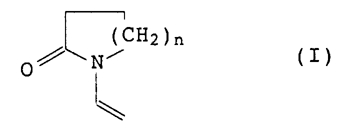 worin n für 1 oder 2 steht, und 60 bis 10 Gew.-%, jeweils bezogen auf die Monomermischung, wenigstens eines hydrophoben Comonomers mit einer Wasserlöslichkeit bei 20°C im Bereich von 1 bis 100 g/l, durch radikalische Polymerisation der Monomere in wässriger Lösung nach einem Zulaufverfahren in Gegenwart eines Initiators, der ausgewählt ist unter Azoinitiatoren, H₂O₂, Hydroperoxiden in Kombination mit Reduktionsmitteln und Persalzen, dadurch gekennzeichnet, daß man den Initiator als Lösung in Wasser und/oder einem C₁-C₄-Alkohol zum Reaktionsansatz gibt, und wobei man die Hauptmenge der Monomere, gegebenenfalls als Lösung in Wasser und/oder einem C₁-C₄-Alkohol, dem Reaktionsansatz so zudosiert, dass während der Polymerisation keine Entmischung im Reaktionsansatz auftritt.

Das erfindungsgemäße Verfahren eignet sich für die Herstellung wasserlöslicher Polymerisate aus Monomermischungen, deren Gehalt an hydrophoben Monomeren im Bereich von 10 bis 60 Gew.-%, vorzugsweise 10 bis 40 Gew.-%, bezogen auf die Monomermischung, liegt. Geeignete hydrophobe Monomere mit einer Wasserlöslichkeit im Bereich von 1 bis 100 g/l sind beispielsweise Vinylacetat, Vinylpropionat, N-Vinylcaprolactam, Methylacrylat, Ethylacrylat, n-Propylacrylat, n-Butylacrylat, t-Butylacrylat, Methylmethacrylat, Ethylmethacrylat, Acrylnitril oder Methacrylnitril.

Als Initiatoren für die radikalische Polymerisation kommen Azoverbindungen in Frage, die für die radikalische Polymerisation in wäßriger Lösung geeignet sind. Besonders geeignet sind aliphatische oder cycloaliphatische Azoverbindungen, z.B. 2,2'-Azobis(isobutyronitril), 2,2'-Azobis(2-methylbutyronitril), 2,2'-Azobis(2,4-dimethylvaleronitril), 1,1'-Azobis(1-cyclohexancarbonitril), 2-(Carbamoylazo)isobutyronitril, 4,4'-Azobis(4-cyanovaleriansäure) und deren Alkalimetall- und Ammoniumsalze, z.B. das Natriumsalz, Dimethyl-2,2'-azobisisobutyrat, 2,2'-Azobis[2-(2-imidazolin-2-yl)propan], 2,2'-Azobis(2-amidinopropan) und die Säureadditionssalze der beiden zuletzt genannten Verbindungen, z.B. die Dihydrochloride. Die in Wasser löslichen Initiatoren sind besonders bevorzugt.

Ferner kommen als Initiatoren Wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen (II)-Salz oder Ascorbinsäure. Geeignete Persalze sind insbesondere Alkalimetallperoxidisulfate.

Die verwendete Initiatormenge, bezogen auf die Monomere, liegt im Bereich von 0,02 bis 15 Mol-%, vorzugsweise 0,05 bis 3 Mol-%. Im erfindungsgemäßen Verfahren wird der Initiator als Lösung, je nach Löslichkeit, in Wasser und/oder in einem C₁-C₄-Alkohol eingesetzt. In diesen Lösungen liegt die Initiatorkonzentration im Bereich von 0,02 bis 2 Mol-%, vorzugsweise 0,1 bis 2 Mol-%, bezogen auf das Lösungsmittel.

Das Polymerisationsmedium ist Wasser, das bis zu 10 Gew.-%, vorzugsweise bis zu 5 Gew.-%, bezogen auf den gesamten Ansatz, eines C₁₋ bis C₄-Alkohols enthalten kann. Das Polymerisationsmedium enthält vorzugsweise nur so viel Alkohol, wie für das Auflösen und Zudosieren der zum Teil nur eingeschränkt in Wasser löslichen Polymerisationsinitiatoren notwendig ist. Besonders bevorzugt wird Wasser als alleiniges Lösungsmittel verwendet. Die Polymerisation wird üblicherweise bei einem neutralen pH-Wert im Bereich von 5 bis 9 durchgeführt. Sofern notwendig, wird der pH-Wert durch Zugabe einer Base, wie Ammoniak, NaOH oder einer Säure, wie HCl, eingestellt bzw. aufrechterhalten.

Beim erfindungsgemäßen Verfahren können die Monomere im wässrigen Lösungsmittel vorgelegt werden (Batch-Verfahren). Vorzugsweise werden jedoch die Monomere, gegebenenfalls als Lösung in Wasser und/oder einem C₁-C₄-Alkohol, dem Reaktionsansatz zudosiert (Zulaufverfahren). Hierbei werden die Monomere so zudosiert, daß keine Entmischung im Reaktionsansatz auftritt, d.h. stets eine klare Lösung vorliegt. Hierdurch wird gewährleistet, daß das hydrophobe Monomer gleichmäßig mit dem wasserlöslichen N-Vinyllactam I abreagieren kann. In einer besonders bevorzugten Ausführungsform werden bis zu 30 Gew.-%, vorzugsweise bis zu 25 Gew.-% des wasserlöslichen N-Vinyllactams I (bezogen auf die Gesamtmenge an N-Vinyllactam I) und eine geringe Menge der Initiatorlösung und Lösungsmittel, vorzugsweise Wasser, vorgelegt. Anschließend wird die Mischung auf Reaktionstemperatur gebracht und die restliche Monomerenmenge gleichzeitig mit der restlichen Initiatorlösung und gegebenenfalls einem Regler, kontinuierlich oder in mehreren Portionen zudosiert. Im allgemeinen erfolgt das Zudosieren über einen Zeitraum von 4 bis 14 Stunden, vorzugsweise 5 bis 12 Stunden, idealerweise 6 bis 10 Stunden. Wird N-Vinylcaprolactam als hydrophobes Monomer eingesetzt, dann arbeitet man vorzugsweise im Batch-Verfahren mit Wasser als alleinigem Lösungsmittel. Die Konzentration der Monomeren im Reaktionsansatz liegt im Bereich von 10 bis 40 Gew.-%, vorzugsweise 15 bis 30 Gew.-%, bezogen auf den Reaktionsansatz. In diesem Fall läßt man die Initiatorlösung, nachdem man den Reaktionsansatz auf die gewünschte Reaktionstemperatur gebracht hat, kontinuierlich oder in mehreren Portionen, insbesondere über einen Zeitraum von 1 bis 4 Stunden, zulaufen.

Die Reaktionstemperatur liegt üblicherweise im Bereich von 60 bis 90°C, sie kann jedoch auch bis zu 130°C betragen. Die Umsetzung kann bei Normaldruck, unter Eigendruck oder unter Schutzgas-Überdruck durchgeführt werden. Als Schutzgas eignet sich beispielsweise Stickstoff. Bei der Reaktionsführung ist zu beachten, daß erhöhte Temperaturen in der Regel zu Polymeren mit kleineren K-Werten, d.h. niedrigeren Molekulargewichten führen.

Die durch das erfindungsgemäße Polymerisationsverfahren erhaltenen Polymerisate weisen in der Regel ein relativ hohes Molekulargewicht auf. Sind niedrigere Molekulargewichte erwünscht, so können diese durch Zusatz eines Reglers zum Polymerisationsansatz eingestellt werden. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten. Hierbei handelt es sich beispielsweise um Din-butylsulfid, Di-n-octylsulfid, Diphenylsulfid, Diisopropyldisulfid, Di-n-butyldisulfid, Di-n-hexyldisulfid, Diacetyldisulfid und Di-t-butyltrisulfid. Vorzugsweise enthalten die Regler Schwefel in Form von SH-Gruppen. Beispiele für solche Regler sind n-Butylmercaptan, n-Hexylmercaptan oder n-Dodecylmercaptan. Besonders bevorzugt werden wasserlösliche, schwefelhaltige Polymerisationsregler, wie beispielsweise Hydrogensulfite, Disulfite und Verbindungen, wie Ethylthioglycolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobernsteinsäure, Thioglycerin, Diethanolsulfid, Thiodiglycol, Ethylthioethanol, Thioharnstoff und Dimethylsulfoxid. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan. In einer bevorzugten Ausführungsform wird der Regler, gegebenenfalls als Lösung in Wasser und/oder einem C₁-C₄-Alkohol dem Reaktionsansatz zudosiert.

Vorzugsweise setzt man der Polymerlösung im Anschluß an die Polymerisationsreaktion einen oder mehrere Polymerisationsinitiatoren zu und erhitzt die Polymerlösung, z.B. auf die Polymerisationstemperatur oder auf Temperaturen oberhalb der Polymerisationstemperatur, um die Polymerisation zu vervollständigen. Geeignet sind die oben angegebenen Azoinitiatoren aber auch alle anderen üblichen, für eine radikalische Polymerisation in wäßriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion bis zu einem Umsatz von 99,9 % geführt. Die bei der Polymerisation entstehenden Lösungen enthalten üblicherweise 10 bis 60 Gew.-%, vorzugsweise 15 bis 40 Gew.-% Polymerisat. Die erhaltenen Lösungen können im Anschluß an die Polymerisation auch einer physikalischen Nachbehandlung, beispielsweise einer Wasserdampf-Destillation oder Strippen mit Stickstoff, unterworfen werden, wobei mit Wasserdampf flüchtige Verunreinigung aus der Lösung entfernt werden.

Die wässrigen Lösungen des Copolymerisats können gegebenenfalls durch ein dem Stand der Technik entsprechenden Trocknungsverfahren in feste Pulver überführt werden. Als Trocknungsverfahren kommen diejenigen in Frage, die zur Trocknung aus wässriger Lösung geeignet sind. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung.

Die erhaltenen Polymerisate besitzen im allgemeinen einen K-Wert (bestimmt bei 25°C in einer 1 Gew.-%igen wäßrigen oder ethanolischen Lösung) im Bereich von 20 bis 100, insbesondere > 40 bis 100 und besonders bevorzugt ≥ 60 bis 100. Die Bestimmung des K-Wertes ist beschrieben in H.Fikentscher "Systematik der Cellulosen auf Grund ihrer Viskosität in Lösung", Cellulose-Chemie 13 (1932), 58-64 und 71-74 sowie Encyclopedia of Chemical Technology, Vol. 21, 2. Ausgabe, 427-428 (1970).

Klar wasserlösliche Polymerisate mit einem K-Wert ≥ 50 waren bisher nicht erhältlich. Gegenstand der Erfindung sind daher auch klar wasserlösliche Copolymerisate, wie oben definiert, mit einem K-Wert ≥ 50.

Als Maß für ihre Wasserlöslichkeit dient die nephelometrische Trübungseinheit FNU (bzw. NTU), die bei 25°C an einer 5 Gew.-%igen wässrigen Lösung des Polymers gemessen und durch Eichung mit Formazin als künstlichem Trübungsmittel festgelegt wird. Die genaue Methode ist im Rahmen der nachfolgenden Beispiele angegeben. Die erfindungsgemäß erhaltenen Polymerisate weisen einen FNU-Wert ≤ 20, insbesondere ≤ 10, vorzugsweise ≤ 7 und besonders ≤ 5 auf.

Die nach dem erfindungsgemäßen Verfahren erhaltenen Polymerisate wirken einerseits in wässrigem Medium als Verdicker und sind andererseits in der Lage, wasserlösliche Filme zu bilden. Sie kommen daher insbesondere zur Anwendung in kosmetischen und pharmazeutischen Zubereitungen, beispielsweise als Additive oder Träger in Haarlack, Haarfestiger oder Haarspray; in hautkosmetischen Zubereitungen, als Hautklebegele oder als Immunochemikalien, z. B. als Katheter-Coatings. Spezielle pharmazeutische Anwendungen der erfindungsgemäßen Polymerisate umfassen insbesondere den Einsatz als Feucht- oder Trockenbindemittel, Matrixretardierungsmittel oder Coatingretardierungsmittel (für Slow-Release-Darreichungsformen), Gelbildner, Instant-Release-Coatings und Dragierhilfsmittel. Weiterhin können die erfindungsgemäß hergestellten Polymerisate als Hilfsstoffe für die Agrochemie, beispielsweise für das Saatgut-Coating oder für Soil-Release-Düngemittelformulierungen oder als Hilfsmittel bei der Herstellung von Fischfuttergranulaten verwendet werden.

Aufgrund der hohen Dispergierwirkung der erfindungsgemäß hergestellten Polymerisate, sowohl für organische als auch für anorganische Pigmente, kommen die erfindungsgemäßen Polymerisate als Rostverhinderer oder Rostentferner von metallischen Oberflächen, als Kesselsteinverhinderer oder Kesselsteinentferner, als Dispergiermittel in Farbstoffpigmentdispersionen, beispielsweise in Druckfarben, in Frage. In diesem Zusammenhang sei auf die Verwendung der erfindungsgemäßen Polymerisate für Tintenstrahl-Aufzeichnungsmedien, Tinten- und Kugelschreiberpasten hingewiesen.

Anwendungstechnisch von Interesse ist weiterhin die hohe Neigung der erfindungsgemäßen Polymerisate, Komplexe mit organischen Verbindungen (z. B. niedere Kohlenwasserstoffe, Phenole, Tannin sowie diverse Antioxydanzien), mit Enzymen und Proteinen, oder mit anderen organischen Polymeren zu bilden. Weiterhin bilden die erfindungsgemäßen Polymerisate Komplexe mit anorganischen Verbindungen, insbesondere mit Wasserstoffperoxid, Halogeniden, Metallen oder Metallsalzen. Dementsprechend können die erfindungsgemäßen Polymerisate zur Entfernung von Tannin, Phenolen, Eiweißstoffen oder polyvalenten Kationen aus wässrigem Medium, in Ionenaustauschern, zur Stabilisierung von Wasserstoffperoxid, beispielsweise in Desinfektionsmitteln, zur Stabilisierung von Antioxydanzien, beispielsweise in Konservierungsmitteln, als polymerer Coligand für Metallkomplexe der reversiblen Sauerstoffabsorption oder für Katalysatoren verwendet werden. Die erfindungsgemäßen Polymerisate können weiterhin für die Stabilisierung von Metallkolloiden verwendet werden. In diesem Zusammenhang sei auch auf die Verwendung der erfindungsgemäßen Polymerisate als Edelmetall-Kristallisationskeime für die Silberfällung und als Stabilisator für Silberhalogenidemulsionen hingewiesen.

Die erfindungsgemäßen Polymerisate eignen sich weiterhin zur Modifizierung von Oberflächen- und Grenzflächeneigenschaften. Sie lassen sich beispielsweise zur Hydrophilisierung von Oberflächen einsetzen und können demnach als Textilhilfsmittel, beispielsweise als Abzieh- und Egalisierungshilfsmittel für die Textileinfärbung, als Aufhellmittel beim Textildruck etc. verwendet werden. Aufgrund der modifizierenden Wirkung für Oberflächen können die erfindungsgemäßen Mittel als Coatings, z. B. für Polyolefine, für Glas und Glasfasern verwendet werden. Aufgrund ihrer oberflächenaktiven Wirkung finden sie weiterhin als Schutzkolloide, beispielsweise bei der Stabilisierung von Metallkolloiden oder bei der radikalischen wässrigen Emulsionspolymerisation Verwendung. In diesem Zusammenhang sei auch auf die Verwendung der erfindungsgemäßen Polymerisate als Hilfsmittel bei der Erdölgewinnung aus ölhaltigem Wasser, als Hilfsmittel bei der Erdöl- und Erdgasförderung sowie dem Erdöl- und Erdgastransport hingewiesen. Weiterhin finden die erfindungsgemäßen Polymerisate Verwendung als Hilfsmittel bei der Reinigung von Abwässern, sei es als Flokkungshilfsmittel oder bei der Entfernung von Farb- und Ölresten aus Abwasser. Weiterhin können die erfindungsgemäßen Polymerisate als Phasentransferkatalysatoren und als Löslichkeitsverbesserer verwendet werden.

Weiterhin finden die erfindungsgemäßen Polymerisate Verwendung bei der Anfärbung von Polyolefinen, als Farbübertragungsinhibitoren, als Farbmischungsinhibitoren für fotografische Diffusions-Transfer-Materialien, als Haftvermittler für Farbstoffe, als Hilfsmittel für die Lithografie, für das Foto-Imaging, für die Diazotypie, als Hilfsmittel für den Metallguß und die Metallhärtung, als Hilfsmittel für Metallquenchbäder, als Hilfsmittel bei der Gasanalytik, als Bestandteil in Keramikbindern, als Papierhilfsmittel für Spezialpapiere, als Bindemittel in Papierstreichfarben und als Bindemittelbestandteil in Gipsbinden.

Die erfindungsgemäßen Polymerisate eignen sich weiterhin als Protonenleiter und können in elektrisch leitenden Schichten, z. B. bei Charge-Transfer-Kathoden, als Festelektrolyte, z. B. in Festbatterien wie Lithiumbatterien, eingesetzt werden. Aus den erfindungsgemäßen Polymerisaten können Kontaktlinsen, synthetische Fasern, Luftfilter, z. B. Zigarettenfilter, oder Membrane hergestellt werden. Weiterhin finden die erfindungsgemäßen Polymerisate Verwendung in wärmebeständigen Schichten, wärmeempfindlichen Schichten und wärmeempfindlichen Widerständen.

Die im folgenden aufgeführten Beispiele sollen die Erfindung verdeutlichen, ohne sie jedoch einzuschränken.

### Beispiele

Die Trübung der wässrigen Copolymerisat-Lösung wurde durch nephelometrische Trübungsmessung bestimmt (modifizierte Methode nach DIN 38404). Bei dieser Methode wird das von der Meßlösung gestreute Licht photometrisch ermittelt, wobei die Lichtstreuung durch die Wechselwirkung zwischen den Lichtstrahlen und den Partikeln oder Tröpfchen in der Lösung, deren Anzahl und Größe den Grad der Trübung ausmachen, verursacht wird. Als Meßgröße dient hierbei die nephelometrische Trübungseinheit FNU (bzw. NTU), die bei 25°C an einer 5 Gew.-%igen wässrigen Lösung des Polymers gemessen und durch Eichung mit Formazin als künstlichem Trübungsmittel festgelegt wird. Je höher der FNU-Wert ist, desto trüber ist die Lösung.

### Beispiel 1

Copolymer aus 70 Gew.-% N-Vinylpyrrolidon und 30 Gew.-% Methylacrylat.

In einem Reaktor mit Rührer, Rückflußkühler, Gaseinlaß und zwei getrennten Zuläufen wurde eine Mischung aus

| | |
|---|---|
| 50 g | N-Vinylpyrrolidon, |
| 5 g | Starterzulauf 1, |
| 1000 g | Wasser |

vorgelegt. Die Vorlage wurde mit Stickstoff gespült und auf 70°C Innentemperatur erwärmt. Anschließend wurden unter Aufrechterhaltung der Temperatur der Monomerzulauf und der Starterzulauf 1 gleichzeitig und mit konstanter Geschwindigkeit über einen Zeitraum von 6 Stunden zugegeben. Anschließend erhöhte man die Innentemperatur auf 75°C und gab den Starterzulauf 2 über einen Zeitraum von 6 Stunden bei Aufrechterhaltung der Temperatur zu. Die Temperatur wurde weitere 2 Stunden bei 75°C gehalten. Anschließend wurde das Reaktionsgemisch einer Wasserdampfdestillation unterworfen und etwa 100 g Destillat wurden aufgefangen. Die Eigenschaften der erhaltenen klaren, viskosen Polymerlösung sind in Tabelle 1 zusammengefaßt.

| Monomerzulauf, bestehend aus | |
|---|---|
| 160 g | N-Vinylpyrrolidon, |
| 90 g | Methylacrylat. |

### Starterzulauf 1,

Lösung von 1 g 2,2'Azobis(2-amidinopropan)dihydrochlorid in 100 g Wasser.

### Starterzulauf 2,

Lösung von 2 g t-Butylperoxypivalat in 40 g Isopropanol.

Der pH-Wert der Vorlage und des Starterzulaufs 1 wurde mit verdünnter Ammoniaklösung auf pH 5 eingestellt.

### Beispiel 2

### Copolymer aus 60 Gew.-% N-Vinylpyrrolidon und 40 Gew.-% Methylacrylat

Es wurde wie in Beispiel 1 verfahren. Die Eigenschaften der erhaltenen klaren, viskosen Polymerlösung sind in Tabelle 1 zusammengefaßt.

| Monomerzulauf, bestehend aus | |
|---|---|
| 130 g | N-Vinylpyrrolidon, |
| 120 g | Methylacrylat. |

### Beispiel 3

### Copolymer aus 70 Gew.-% N-Vinylpyrrolidon und 30 Gew.-% Ethylacrylat

Es wurde wie in Beispiel 1 verfahren. Die Eigenschaften der erhaltenen klaren, viskosen Polymerlösung sind in Tabelle 1 zusammengefaßt.

| Monomerzulauf, bestehend aus | |
|---|---|
| 160 g | N-Vinylpyrrolidon, |
| 90 g | Ethylacrylat. |

### Beispiel 4

### Copolymer aus N-Vinylpyrrolidon und Methylmethacrylat 80:20

Es wurden zunächst die folgenden Lösungen vorbereitet:
1. Vorlage, bestehend aus 1000 g Wasser, 50 g N-Vinylpyrrolidon und 5 g Starterzulauf 1
2. Monomerzulauf, bestehend aus 190 g N-Vinylpyrrolidon und 60 g Methylmethacrylat.
3. Starterzulauf 1, bestehend aus 1 g 2,2'-Azobis(2-amidinopropan)dihydrochlorid, gelöst in 100 g Wasser.
4. Starterzulauf 2, bestehend aus 1 g 2,2'-Azobis(2-amidinopropan)dihydrochlorid, gelöst in 100 g Wasser.
Die Fahrweise, die Polymerisationsdauer und -temperatur und der eingestellte pH-Wert entsprechen denen in Beispiel 1. Bereits gegen Ende der Polymerisation, aber besonders während der Wasserdampfdestillation wurde das Polymer unlöslich (Ausfällung und Verklumpung), beim Abkühlen entstand jedoch eine klare und viskose wäßrige Lösung des Polymeren.

Die erhaltene farblose, viskose Lösung des Polymeren wies einen Feststoffgehalt von 19,4 Gew.-% auf und der K-Wert des Produktes (gemessen 1%ig in Wasser) lag bei 79,1. Als Verunreinigungen wurden < 0,005% N-Vinylpyrrolidon und 0,1% Pyrrolidon gefunden. Der FNU-Wert der 5 gew.-%igen Lösung in Wasser lag bei 1,5.

### Beispiel 5

Copolymer aus 70 Gew.-% N-Vinylpyrrolidon und 30 Gew.-% Vinylacetat.

In einem Reaktor mit Rührer, Rückflußkühler, Gaseinlaß und zwei getrennten Zuläufen wurde eine Mischung aus
100 g N-Vinylpyrrolidon,
0.2 g 2,2'-Azobis(2-amidinopropan)dihydrochlorid,
2 g Ammoniakwasser (25 %),
1000 g Wasser
vorgelegt. Die Vorlage wurde mit Stickstoff gespült und auf 70°C Innentemperatur erwärmt. Anschließend gab man gleichzeitig mit konstanter Geschwindigkeit Monomerzulauf 1 innerhalb von 5 Stunden und Starterzulauf 1 innerhalb von 8 Stunden unter Aufrechterhaltung der Temperatur zum Reaktionsansatz. Nach Beendigung des Monomerzulaufs 1 wurde unmittelbar im Anschluß Monomerzulauf 2 über einen Zeitraum von 2,5 Stunden mit konstanter Geschwindigkeit zugegeben. Anschließend wurde eine weitere Stunde bei 70°C Innentemperatur nachpolymerisiert. Dann wurde die Temperatur auf 75°C erhöht und der Starterzulauf 2 über einen Zeitraum von 6 Stunden mit konstanter Geschwindigkeit unter Aufrechterhaltung der Innentemperatur zugegeben. Nach Beendigung der Zugabe wurde unter Rühren die Temperatur noch weitere 2 Stunden beibehalten. Anschließend wurde der Reaktionsansatz einer Wasserdampfdestillation unterworfen und 100 g Destillat aufgefangen.

Die Eigenschaften der klaren, viskosen Polymerlösung sind in Tabelle 1 zusammengefaßt.

| Monomerzulauf 1, bestehend aus | |
|---|---|
| 450 g | N-Vinylpyrrolidon |
| 300 g | Vinylacetat |
| 5 g | Ammoniakwasser |

| Monomerzulauf 2, bestehend aus | |
|---|---|
| 150 g | N-Vinylpyrrolidon |
| 250 g | Wasser |

### Starterzulauf 1,

### Lösung von 2 g 2,2'-Azobis(2-amidinopropan)dihydrochlorid in 100 g Wasser

### Starterzulauf 2,

### Lösung von 2 g 2,2'-Azobis(2-amidinopropan)dihydrochlorid in 150 g Wasser

### Beispiel 6

### Copolymer aus N-Vinylpyrrolidon und Vinylacetat 70:30 (geregelte Fahrweise)

Es wurden zunächst die folgenden Lösungen vorbereitet:
1. Vorlage, bestehend aus 1500 g Wasser, 100 g N-Vinylpyrrolidon, 0,2 g 2,2'-Azobis(2-amidinopropan)dihydrochlorid und 2 g Ammoniakwasser (25%).
2. Monomerzulauf 1, bestehend aus 450 g N-Vinylpyrrolidon, 300 g Vinylacetat, 3 g Mercaptoethanol und 5 g Ammoniakwasser (25%).
3. Starterzulauf 1, bestehend aus 5 g 2,2'-Azobis(2-amidinopropan)dihydrochlorid, gelöst in 150 g Wasser.
4. Monomerzulauf 2, bestehend aus 150 g N-Vinylpyrrolidon, 1,9 g Mercaptoethanol und 250 g Wasser.
5. Starterzulauf 2, bestehend aus 2 g 2,2'-Azobis(2-amidinopropan)dihydrochlorid, gelöst in 100 g Wasser.
Die Fahrweise, die Polymerisationsdauer und -temperatur und die Wasserdampfdestillation entsprechen denen in Beispiel 5.

Die erhaltene farblose, niederviskose Lösung des Polymeren wies einen Feststoffgehalt von 28,5 Gew.-% auf und der K-Wert des Produktes (gemessen 1%ig in Ethanol) lag bei 37,7. Als Verunreinigungen wurden 0,005% N-Vinylpyrrolidon und 0,12% Pyrrolidon gefunden. Der FNU-Wert der 5 gew.%igen Lösung in Wasser lag bei 4,0.

### Beispiel 9

### Copolymer aus 50 Gew.-% N-Vinylpyrrolidon und 50 Gew.-% N-Vinylcaprolactam, geregelte Fahrweise

In einem Reaktor mit Rührer, Rückflußkühler, Gaseinlaß und drei getrennten Zuläufen legte man 1000 g Wasser vor, spülte die Vorlage mit Stickstoff und erwärmte auf 80°C Innentemperatur. Dann wurden der Monomerzulauf, der Starterzulauf 1 und der Reglerzulauf 1 gleichzeitig innerhalb von 3 Stunden mit konstanter Geschwindigkeit der Reaktionsmischung zugefügt. Anschließend wurde die Reaktionstemperatur auf 80°C angehoben und Starterzulauf 2 sowie Reglerzulauf 2 unter Aufrechterhaltung dieser Temperatur innerhalb von 2 Stunden mit konstanter Geschwindigkeit der Reaktionsmischung zugefügt. Die Temperatur wurde auf 85°C angehoben und für 3 weitere Stunden beibehalten. Die Eigenschaften der erhaltenen niederviskosen Polymerlösung sind in Tabelle 2 zusammengefaßt.

| Monomerzulauf, bestehend aus | |
|---|---|
| 150 g | N-Vinylpyrrolidon, |
| 150 g | N-Vinylcaprolactam. |

### Starterzulauf 1,

Lösung von
3 g 2,2'-Azobis(2-amidinopropan)dihydrochlorid in 60 g Wasser.

### Starterzulauf 2,

Lösung von
1,8 g 2,2'-Azobis(2-amidinopropan)dihydrochlorid in 60 g Wasser.

| Reglerzulauf 1, bestehend aus | |
|---|---|
| 3 g | Mercaptoethanol, |
| 30 g | Wasser. |

| Reglerzulauf 2, bestehend aus | |
|---|---|
| 1,2 g | Mercaptoethanol, |
| 30 g | Wasser. |

Die Reglerzuläufe wurden mit verdünnter Natronlauge auf pH 6 eingestellt.

**Tabelle 1**

| | Beispiel 1 | Beispiel 2 | Beispiel 3 | Beispiel 5 |
|---|---|---|---|---|
| Feststoffgehalt | 19,7 Gew.-% | 19,9 Gew.-% | 18,7 Gew.-% | 35,7 Gew.-% |
| | | | | |
| K-Wert | 73,4 | 69,5 | 71,5 | 62,0* |
| | | | | |
| FNU | 3,4 | 5.2 | 6,7 | 3,5 |
| | | | | |
| Farbe | farblos | farblos | farblos | farblos |
| Verunreinigung | | | | |
| | | | | |
| N-Vinylpyrrolidon | 0,005 Gew-% | 0,006 Gew-% | 0,01 Gew-% | 0,007 Gew-% |
| | | | | |
| Pyrrolidon | 0,087 Gew-% | 0,117 Gew-% | 0,03 Gew-% | 0,11 Gew-% |

| | | | | |
|---|---|---|---|---|
| * 1 %ig in Ethanol | | | | |

**Tabelle 2**

| | Beispiel 9 |
|---|---|
| Feststoffgehalt | 29,3 Gew.-% |
| | |
| K-Wert | 23,0 |
| | |
| FNU | 3,5 |
| | |
| Farbe | gelblich |
| Verunreinigung | |
| | |
| N-Vinylpyrrolidon | 0,015 Gew.-% |
| | |
| N-Vinylcaprolactam | < 0,005 Gew.-% |

### Vergleichsbeispiel 1

Es wurde das Beispiel 1 der DE-A-2218935 wiederholt. Man erhält eine inhomogene, teilweise koagulierte, viskose Polymerlösung.

### Vergleichsbeispiel 2

Es wurde das Beispiel 3 der DE-A-2218935 wiederholt. Man erhält eine niederviskose, unvollständig polymerisierte und stark trübe Polymerlösung (FNU > 200).

## Patentansprüche

1. Verfahren zur Herstellung klar wasserlöslicher Copolymerisate, die aufgebaut sind aus 40 bis 90 Gew.-% wenigstens eines wasserlöslichen N-Vinyllactams der allgemeinen Formel I, 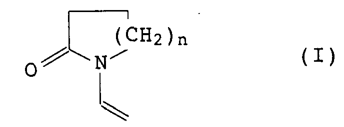 worin n für 1 oder 2 steht, und 60 bis 10 Gew.-%, jeweils bezogen auf die Monomermischung, wenigstens eines hydrophoben Comonomers mit einer Wasserlöslichkeit bei 20°C im Bereich von 1 bis 100 g/l, durch radikalische Polymerisation der Monomere in wässriger Lösung nach einem Zulaufverfahren in Gegenwart eines Initiators, der ausgewählt ist unter Azoinitiatoren, H₂O₂, Hydroperoxiden in Kombination mit Reduktionsmitteln und Persalzen, **dadurch gekennzeichnet, daß** man den Initiator als Lösung in Wasser und/oder einem C₁-C₄-Alkohol zum Reaktionsansatz gibt, und wobei man die Hauptmenge der Monomere, gegebenenfalls als Lösung in Wasser und/oder einem C₁-C₄-Alkohol, dem Reaktionsansatz so zudosiert, dass während der Polymerisation keine Entmischung im Reaktionsansatz auftritt.

2. Verfahren nach Anspruch 1, **dadurch gekennzeichnet, daß** man 10 bis 40 Gew.-%, bezogen auf das Gesamtgewicht der Monomere, des hydrophoben Comonomers verwendet.

3. Verfahren nach Anspruch 1 oder 2, **dadurch gekennzeichnet, daß** man als hydrophobes Comonomer Vinylacetat, Vinylpropionat, N-Vinylcaprolactam, Methylacrylat, Ethylacrylat, n-Propylacrylat, n-Butylacrylat, t-Butylacrylat, Methylmethacrylat, Ethylmethacrylat, Acrylnitril, Methacrylnitril oder deren Mischungen verwendet.

4. Verfahren nach einem der vorhergehenden Ansprüche, **dadurch gekennzeichnet, daß** man als Initiator eine aliphatische oder cycloaliphatische Azoverbindung verwendet.

5. Verfahren nach Anspruch 4, **dadurch gekennzeichnet, daß** man als Initiator 2,2'-Azobis[2-(2-imidazolin-2-yl)propan], 2,2'-Azobis(2-amidinopropan), 4,4'-Azobis(4-cyanovaleriansäure) oder ein Salz dieser Verbindungen, 2,2'-Azobis(isobutyramid), 2,2'-Azobis(isobutyronitril), 2,2'-Azobis(2-methylbutyronitril), Dimethyl-2,2'-azobisisobutyrat, 2,2'-Azobis(4-methoxy-2,4-dimethylvaleronitril), 2,2'-Azobis(2,4-dimethylvaleronitril) und/oder 1,1'-Azobis(1-cyclohexancarbonitril) verwendet.

6. Verfahren nach einem der vorhergehenden Ansprüche, **dadurch gekennzeichnet, daß** man die Polymerisationsreaktion in Wasser, das gegebenenfalls bis zu 10, vorzugsweise bis zu 5 Gew.-% eines C₁-C₄-Alkohols enthalten kann, durchführt.

7. Verfahren nach einem der vorhergehenden Ansprüche, **dadurch gekennzeichnet, daß** man die Polymerisation in Anwesenheit eines Reglers durchführt.

8. Klar wasserlösliche Copolymerisate mit einem K-Wert von ≥50, die aufgebaut sind aus 40 bis 90 Gew.-% wenigstens eines wasserlöslichen N-Vinyllactams der allgemeinen Formel I 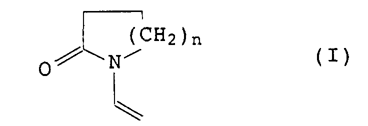 worin n für 1 oder 2 steht, und 60 bis 10 Gew.-%, jeweils bezogen auf das Gesamtgewicht der Monomere, wenigstens eines hydrophoben Comonomers mit einer Wasserlöslichkeit bei 20°C im Bereich von 1 bis 100 g/l.

9. Copolymerisat nach Anspruch 8, wobei es sich bei dem hydrophoben Comonomer um Vinylacetat, Vinylpropionat, N-Vinylcaprolactam, Methylacrylat, Ethylacrylat, n-Propylacrylat, n-Butylacrylat, t-Butylacrylat, Methylmethacrylat, Ethylmethacrylat, Acrylnitril, Methacrylnitril oder deren Mischungen handelt.

10. Copolymerisate nach einem der Ansprüche 8 oder 9 mit einem FNU-Wert ≤ 20.

11. Verwendung der Copolymerisate nach einem der Ansprüche 8 bis 10 in kosmetischen oder pharmazeutischen Zubereitungen und als Hilfsstoffe in der Agrochemie.

## Claims

1. A process for preparing copolymers which can be dissolved in water to give clear solutions and are synthesized from from 40 to 90% by weight of at least one water-soluble N-vinyllactam of the formula I 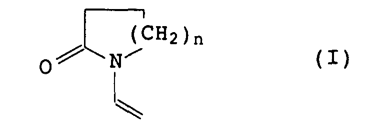 in which n is 1 or 2 and from 60 to 10% by weight, based in each case on the monomer mixture, of at least one hydrophobic comonomer having a water-solubility of from 1 to 100 g/l at 20°C by free-radical polymerization of the monomers in aqueous solution by a feed process in the presence of an initiator selected from azo initiators, H₂O₂, hydroperoxides in combination with reducing agents and persalts, which comprises adding the initiator in the form of a solution in water and/or in a C₁-C₄ alcohol to the reaction mixture and metering the major proportion of the monomers, where appropriate in the form of a solution in water and/or in a C₁-C₄ alcohol, into the reaction mixture in such a way that no separation occurs in the reaction mixture during the polymerization.

2. A process as claimed in claim 1, wherein 10 to 40% by weight, based on the overall weight of the monomers, of the hydrophobic comonomer is used.

3. A process as claimed in claim 1 or 2, wherein the hydrophobic comonomer used is vinyl acetate, vinylpropionate, N-vinylcaprolactam, methyl acrylate, ethyl acrylate, n-propyl acrylate, n-butyl acrylate, t-butyl acrylate, methyl methacrylate, ethyl methacrylate, acrylonitrile, methacrylonitrile or a mixture thereof.

4. A process as claimed in any of the preceding claims, wherein the initiator used is an aliphatic or cycloaliphatic azo compound.

5. A process as claimed in claim 4, wherein the initiator used is 2,2'-azobis[2-(2-imidazolin-2-yl)propane], 2,2'-azobis(2-amidinopropane), 4,4'-azobis(4-cyanovaleric acid) or a salt thereof, 2,2'-azobis(isobutyramide), 2,2'-azobis(isobutyronitrile), 2,2'-azobis(2-methylbutyronitrile), dimethyl 2,2'-azobisisobutyrate, 2,2'-azobis(4-methoxy-2,4-dimethylvaleronitrile), 2,2'-azobis(2,4-dimethylvaleronitrile) and/or 1,1'-azobis(1-cyclohexanecarbonitrile).

6. A process as claimed in any of the preceding claims, wherein the polymerization reaction is carried out in water which may contain up to 10% by weight, preferably up to 5% by weight, of a C₁-C₄-alcohol.

7. A process as claimed in any of the preceding claims, wherein the polymerization is carried out in the presence of a regulator.

8. A copolymer which can be dissolved in water to give a clear solution, has a K value of ≥ 50 and is synthesized from from 40 to 90% by weight of at least one water-soluble N-vinyllactam of the formula I 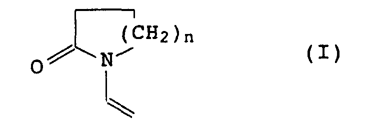 in which n is 1 or 2 and from 60 to 10% by weight, based in each case on the overall weight of the monomers, of at least one hydrophobic comonomer having a water-solubility of from 1 to 100 g/l at 20°C.

9. A copolymer as claimed in claim 8, where the hydrophobic comonomer is vinyl acetate, vinylpropionate, N-vinylcaprolactam, methyl acrylate, ethyl acrylate, n-propyl acrylate, n-butyl acrylate, t-butyl acrylate, methyl methacrylate, ethyl methacrylate, acrylonitrile, methacrylonitrile or a mixture thereof.

10. A copolymer as claimed in either of claims 8 and 9, having an FNU value ≤ 20.

11. The use of a copolymer as claimed in any of claims 8 to 10 in a cosmetic or pharmaceutical preparation or as an agrochemical auxiliary.

## Revendications

1. Procédé pour la préparation de copolymères hydrosolubles limpides, qui sont élaborés à partir de 40 à 90 % en poids d'au moins un N-vinyllactame hydrosoluble de formule générale I, 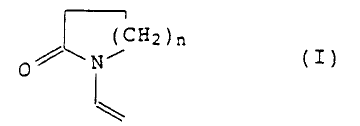 où n vaut 1 ou 2, et de 60 à 10 % en poids, à chaque fois par rapport au mélange de monomères, d'au moins un comonomère hydrophobe ayant une solubilité dans l'eau à 20°C dans l'intervalle de 1 à 100 g/l, par polymérisation radicalaire des monomères en solution aqueuse selon un procédé par flux en présence d'un initiateur, qui est choisi parmi les initiateurs azoiques, H₂O₂, les hydroperoxydes en combinaison avec des agents réducteurs, et les persels, **caractérisé par le fait qu'**on ajoute l'initiateur sous forme de solution dans l'eau et/ou un alcool en C₁-C₄ à la charge réactionnelle, et tandis qu'on ajoute la majeure partie des monomères, éventuellement sous forme de solution dans l'eau et/ou un alcool en C₁-C₄, à la charge réactionnelle en quantité dosée de telle façon qu'il ne se produise aucune démixtion dans la charge réactionnelle pendant la polymérisation.

2. Procédé selon la revendication 1, **caractérisé par le fait qu'**on utilise 10 à 40 % en poids, par rapport au poids total des monomères, du comonomére hydrophobe.

3. Procédé selon la revendication 1 ou 2, **caractérisé par le fait qu'**on utilise comme comonomère hydrophobe l'acétate de vinyle, le propionate de vinyle, le N-vinylcaprolactame, l'acrylate de méthyle, l'acrylate d'éthyle, l'acrylate de n-propyle, l'acrylate de n-butyle, l'acrylate de t-butyle, le méthacrylate de méthyle, le méthacrylate d'éthyle, l'acrylonitrile, le méthacrylonitrile ou leurs mélanges.

4. Procédé selon l'une des revendications précédentes, **caractérisé par le fait qu'**on utilise comme initiateur un composé azoique aliphatique ou cycloaliphatique.

5. Procédé selon la revendication 4, **caractérisé par le fait qu'**on utilise comme initiateur le 2,2'-azobis[2-(2-imidazoline-2-yl)propane], le 2,2'-azobis(2-amidinopropane), l'acide 4,4'-azobis(4-cyanovalérique) ou un sel de ces composés, le 2,2'-azobis(isobutyr-amide), le 2,2'-azobis(isobutyronitrile), le 2,2'-azobis(2-méthylbutyronitrile), le diméthyl-2,2'-azobisisobutyrate, le 2,2'-azobis(4-méthoxy-2,4-diméthylvaléronitrile), le 2,2'-azobis(2,4-diméthylvaléronitrile) et/ou le 1,1'-azobis(1-cyclohexanecarbonitrile).

6. Procédé selon l'une des revendications précédentes, **caractérisé par le fait qu'**on effectue la réaction de polymérisation dans de l'eau, qui peut éventuellement contenir jusqu'à 10, de préférence jusqu'à 5 % en poids d'un alcool en C₁-C₄.

7. Procédé selon l'une des revendications précédentes, **caractérisé par le fait qu'**on réalise la polymérisation en présence d'un régulateur.

8. Copolymères hydrosolubles limpides ayant un indice K de ≥ 50, qui sont élaborés à partir de 40 à 90 % en poids d'au moins un N-vinyllactame hydrosoluble de formule générale I, 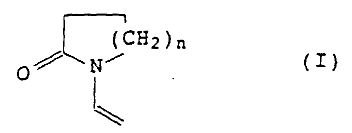 où n vaut 1 ou 2, et de 60 à 10 % en poids, à chaque fois par rapport au poids total des monomères, d'au moins un comonomére hydrophobe ayant une solubilité dans l'eau à 20°C dans l'intervalle de 1 à 100 g/l.

9. Copolymère selon la revendication 8, tandis que pour le comonomère hydrophobe il s'agit d'acétate de vinyle, de propionate de vinyle, de N-vinylcaprolactame, d'acrylate de méthyle, d'acrylate d'éthyle, d'acrylate de n-propyle, d'acrylate de n-butyle, d'acrylate de t-butyle, de méthacrylate de méthyle, de méthacrylate d'éthyle, d'acrylonitrile, de méthacrylonitrile ou de leurs mélanges.

10. Copolymères selon l'une des revendications 8 ou 9 ayant un indice FNU (ou NTU) ≤ 20.

11. Utilisation des copolymères selon l'une des revendications 8 à 10 dans des préparations cosmétiques ou pharmaceutiques et comme adjuvants en agrochimie.
